# NEUE EUROPÄISCHE PATENTSCHRIFT

(11) **EP 1 013 233 B2**
(45) Veröffentlichungstag und Bekanntmachung des Hinweises auf die Entscheidung über den Einspruch: **01.09.2004**
(45) Hinweis auf die Patenterteilung: 24.10.2001
(21) Anmeldenummer: 99125542.3
(22) Anmeldetag: 21.12.1999
(51) Int. Cl.: A61B 17/92

(54) **Vorrichtung zum Eintreiben eines Drahtstiftes, insbesondere eines Kirschnerdrahts in Knochenmaterial**
Device for driving a wire pin, in particular a Kirschner wire, into bone
Dispositif pour l'enfoncement d'une pointe, en particulier une broche de Kirschner, dans un matériau osseux

(30) Priorität: 21.12.1998 DE 19859135
(43) Veröffentlichungstag der Anmeldung: 28.06.2000
(73) Patentinhaber: Ferton Holding SA, 2800 Delémont (CH)
(72) Erfinder: Jerger, Thomas, Dr., 71065 Sindelfingen (DE); van Egmond, Dirk Bernard, Dr., 3445 AS Woerden (NL); Raabe, Thorsten, Dr., 88682 Buggensegel (DE)
(74) Vertreter: Viering, Jentschura & Partner

(56) Entgegenhaltungen:
- EP-B- 0 317 507
- WO-A-88/02246
- WO-A-95/22934
- WO-A-96/33661
- WO-A-97/48353
- DE-A- 2 537 815
- DE-A- 2 735 563
- DE-A- 19 527 529
- DE-A- 19 618 972
- FR-A- 1 548 215
- FR-A- 2 361 089
- SU-A- 128 109

## Beschreibung

Die Erfindung betrifft die Kombination einer Vorrichtung zum Eintreiben eines langgestreckten Drahtstifts, insbesondere eines Kirschnerdrahts, in ein Knochenmaterial, und dem langgestreckten Drahtstift, die Vorrichtung versehen mit einer an dem Drahtstift, der in die Vorrichtung vom vorderen freien Vorrichtungsende her einschiebbar ist, angreifenden Vortriebsvorrichtung, die von einem Anschlagstöße erzeugenden Hammerteil angetrieben ist, das seinerseits periodisch angetrieben ist.

Aus der DE 195 27 529 A1 ist ein medizinisches Werkzeug zum Eintreiben eines kurzen Kopfnagels In ein Knochenmaterial bekannt. Die Vortriebsvorrichtung ist als von einer Rückstellfeder belasteter Übertragungskolben ausgebildet, dervon einem Antriebskolben translatorisch angetrieben wird, der seinerseits in einem Antriebsrohr pneumatisch angetrieben wird, bis er auf den Übertragungskolben auftrifft. Der Nagel wird mit seinem zylindrischen langgestreckten Kopf vom vorderen Vorrichtungsende her in eine Führungsbohrung eingeschoben; bis der Kopf an einem Zapfen des Übertragungskolbens anliegt. Durch den Anschlagstoß, der von dem Antriebskolben auf den Übertragungskolben ausgeübtwird, wird der Nagel translatorisch in das Knochenmaterial hineingedrückt.

Vorrichtungen zum Eintreiben eines Kirschnerdrahtes werden in der Chirurgie, insbesondere zur Behandlung von Knochenbrüchen verwendet. Der Kirschnerdraht, d.h. ein langgestreckter Drahtstift aus z.B. Metall, kann einfach und schnell in Knochenteile eingetrieben werden, ohne daß um diese herum vorliegendes weicheres Gewebe in großem Maße geschädigt wird.

Vorrichtungen dieser Art sind beispielsweise aus der EP 0 597 547 A und aus der DE 27 35 563 A bekannt. Sowohl nach der EP 0 597 547 als auch nach der DE 27 35 563 werden die von dem Hammerteil ausgeübten Anschlagstöße über eine Aufnahmehülse und eine Klemmvorrichtung auf den Drahtstift übertragen. Bei der Vorrichtung nach der EP 0 597 547 wird der Drahtstift vom hinteren Ende der Vorrichtung her durch diese hindurchgeschoben und die Stöße werden über eine Exzenterklemmvorrichtung in Form von Vibrationsschwingungen derart auf den Drahtstift übertragen, daß dieser in schwingender Weise translatorisch vorgetrieben wird. Nach der DE 27 35 563 wird der Draht, der vom vorderen Ende der Vorrichtung in diese eingeschoben und dann festgeklemmt wird, nach dem Prinzip einer Schlagbohrmaschine bei jedem Anschlagstoß zusammen mit der Aufnahmehülse translatorisch jeweils ein Stück vorgetrieben, wonach die restliche Vorrichtung nachgerückt wird. Aufgrund der vorgesehener translatorischen Bewegung des Drahtstifts ist es bei beiden vorstehend erläuterten Vorrichtungen gemäß derr Stand der Technik vorgesehen, die Vorrichtung mög. lichst nahe an dem Knochenmaterial anzuordnen, um die Gefahr eines Knickens des Drahtes zu reduzieren. Um trotzdem eine ausreichende Länge an Draht in das Knochenmaterial eintreiben zu können, ist die jeweilige Klemmvorrichtung als Klemmgesperre ausgebildet, weiches ein Bewegen des Drahtstifts aus der Vorrichtung heraus zuläßt, jedoch ein Bewegen des Drahtstifts in die Vorrichtung hinein unterbindet. Von der jeweiligen Vorrichtung kann somit ein ausreichender Drahtvorrat aufgenommen werden, der nach und nach aus der Vorrichtung heraus in das Knochenmaterial eintreibbar ist. Diese herkömmlichen Vorrichtungen erfordern zum einen aufgrund der relativ aufwendigen Klemm- oder Greifvorrichtungen einen hohen baulichen Aufwand und zum anderen einen erhöhten Energieverbrauch, da die gesamte Klemmvorrichtung mitbeaufschlagt und translatorisch mitveriagert wird. Der Energieverbrauch ist auch deshalb erhöht, weil die Klemmvorichtung starken mechanischen Belastungen ausgesetzt ist und demnach stabil ausgelegt sein muß. Ferner kann auf der Drahtstift seinerseits auch nur ein kleiner Impuls übertragen werden.

Durch die Erfindung wird eine in eingangs erwähnter Art ausgebildete Kombination mit einer Vorrichtung zum Eintreiben eines Drahtstifts, insbesondere eines Kirschnerdrahts, in Knochenmaterial mit einem unkomplizierten Aufbau und geringem Energieverbrauch geschaffen.

Dies wird mit einer Kombination aus einer Vorrichtung zum Eintreiben eines langgestreckten Drahtstiftes in ein Knochenmaterial und dem langgestreckten Drahtstift, mit den Merkmalen im Anspruch 1 erreicht.

Bei der Vorrichtung greift die Vortriebsvorrichtung an dem Drahtstift klemmittelfrei an. Hierzu ist die Vortriebsvorrichtung als elastische Stoßwellen übertragendes Übertragungsstück ausgebildet, an das der Drahtstift mit seinem freien Stirnende in der Führungshülse stumpf angesetzt werden kann. Von dem Übertragungsstück sind ferner die Anschiagstöße des Hammerteils in Form von periodisch wiederholten Stoßimpulsen durch die freie Stirnfläche des Drahtstifts hindurch in diesen einleitbar. Der jeweilige in den Drahtstift eingeleitete Stoßimpuls pflanzt sich in dem Drahtstift in Form einer Kompressionswelle fort, wodurch das dem Knochenmaterial zugewandte Ende des Drahtstifts in das Knochenmaterial hinein ausgelenkt wird und in diesem stecken bleibt, so daß derrestliche Teil des Drahtstifts beim Zurücklaufen der Kompresslonswelle nachgeführt wird. Durch die periodisch aufeinander folgen den Kompressionswellen wird somit der aus dem vorderen freien Vorrichtungsende herausragende Drahtstift periodisch fortschreitend in das Knochenmaterial eingetrieben. Die Vorrichtung wird nach jedem Stoßimpuls nachgeschoben, bis der Drahtstift wieder an dem Übertragungsstück anschlägt und. für den folgenden Vortreibvorgang wieder bereitsteht.

Im Gegensatz zu herkömmlichen Vorrichtungen erfolgt demnach das Eintreiben des Drahtstiftes durch den fortlaufenden Stoßimpuls und nicht durch die translatorische Bewegung des Drahstifts insgesamt. Ferner wird bei der erfindungsgemäßen Vorrichtung der Drahtstift bei der Stoßübertragung nicht an der Vortriebsvorrichtung festgehalten, so daß konstruktiv aufwendige Einrichtungen zum automatischen Festklemmen und Loslassen des Drahts entfallen. Stattdessen liegt der Draht mit seinem Stirnende stets lose an dem Übertragungsstück an.

Bei den herkömmlichen Vorrichtungen ist, wie aus obigen Ausführungen ersichtlich, in der Vorrichtung ein Drahtvorrat aufgenommen, der nach und nach aus der Vorrichtung heraustransportiert wird. Hierzu greift die Vortriebsvorrichtung im zuge des Eintreibvorgangs an unterschiedlichen Abschnitten längs des einzutreibenden Drahtstifts an. Im Gegensatz dazu greift bei der erfindungsgemäßen Vorrichtung die Vortriebsvorrichtung stets lediglich an dem von dem Knochenmaterial abgewandten Stirnende des Drahtstifts an. Hierbei ist jedoch auch bei einem langgestreckten, evtl. leicht gekrümmten Drahtstift, wie einem Kirschnerdraht, die Gefahr eines Knickens des Drahtsstifts nicht gegeben, da dieser nicht durch eine insgesamt translatorische Stoßbewegung in das Knochenmaterial hineinbewegt wird. Dererfindungsgemäß in den Drahtstift eingeleitete Stoßimpuls pflanzt sich als Kompressionswelle auch in einem langgestreckten, gekrümmten Drahtstift fort und bewirkt auch bei leichter Drahtkrümmung eine Auslenkung des knochenmaterialnahen Endes des Drahtstifts in das Knochenmaterial hinein. Der Hub, um den der Drahtstift bei jedem Stoßimpuls in das Knochenmaterial getrieben wird beträgt ca. 1 mm.

Bei der erfindungsgemäßen Vorrichtung dient das Übertragungsstück der Übertragung eines Stoßimpulses auf den Drahtstift und erfährt selbst keine oder nur eine geringfügige translatorische Bewegung. Damit wird keine Energie zur Beschleunigung und Bewegung einer Klemmvorrichtung verbraucht, so daß der Energiebedarf verringert wird. Von dem Stoßimpuls wird In dem Drahtstift, der als Wellenleiter zum Welterlelten von elastischen Längswellen ausgebildet ist, eine solche Längs- oder Kompressionswelle ausgelöst, die sich In dem Drahtstift fortpflanzt und zum örtlichen Expansion und Kompression des Drahtstiftmaterials führt. Das freie Drahtende wird daher in das Knochenmaterial hinein durch das Eintreffen der elastischen Welle ausgelenkt, die dabei in abgeschwächter Form am freien Ende des Drahtstiftes reflektiert wird, so daß sich der freie Drahtabschnitt beim Zurücklaufen der Welle wieder zusammenzieht. Da der freie Endabschnitt jedoch durch sein Zusammenziehen nicht aus dem Knochenmaterial wieder herausgezogen werden kann, wird stattdessen der restliche Teil des Drahtes nachgezogen.

Aus der Lithotripsie ist es an sich bekannt, Stoßimpulse auf einen langgestreckten, drahtförmigen Wellenleiter zu übertragen, um damit z.B. Nierensteine zu zertrümmern. Die DE 196 18 972 A1 offenbart beispielsweise eine Vorrichtung mit einer an einem Wellenleiter angreifenden Schlagübertragungsvorrichtung auf die von einem Hammerteil in periodischer Wiederholung Anschlagstöße ausgeübt werden. Diese bekannte Vorrichtung ist jedoch nicht zum Eintreiben eines Drahtstifts in Knochenmaterial geeignet, da der zur Steinzertrümmerung verwendete drahtförmige Wellenleiter in der Vorrichtung festgehalten ist.

Die erfindungsgemäße Vorrichtung weist eine Führungshülse auf, in deren Führungsbohrung der kopflose Drahtstift bis zum Anschlagen an dem Übertragungsstück frei längsverschiebbar aufgenommen und geführt wird. Der Drahtstift wird dann einfach in die Führungsbohrung eingeschoben und bis zum Anschlagen an dem Übertragungsstück in die Führungshülse hineinbewegt. Beim Bedienen der Vorrichtung muß diese dann lediglich mit dem darin eingesetzten Draht gegen das Knochenmaterial gedrückt gehalten werden. Mit der Führungshülse kann ferner eine wesentlich genauere Führung des Drahtstifts erreicht werden. Die Führungshülse Ist insbesondere derart an der Vorrichtung vorgesehen, daß bei deren Gebrauch der der Vorrichtung zugewandte Endabschnitt des Drahtstifts in die Führungshülse eingesetzt ist. Hierdurch kann der Draht genau parallel zur Stoßübertragungsrichtung an das Übertragungsstück herangeführt werden, wodurch Energieverluste bei der Stoßübertragung auf den Drahtstift verhindert werden. Die Führungshülse verhindert dann ferner auch ein leichtes Knicken des Drahtstifts bei der Stoßübertragung sowie hiermit verbundene Energieverluste. Damit der Stoß von dem Übertragungsstück lediglich auf den Drahtstift und nicht auch auf die Führungshülse-übertragen wird, steht das Übertragungsstück mit der Führungshülse nicht in einem stoßübertragenden Kontakt.

Das Übertragungsstück kann irgendein Bauteil sein, z.B. ein Kipphebel oder eine den Drahtstift umgebende, einseitig offene Dose, und ist vorzugsweise aus einem festen Material, in dem elastische Stoßwellen übertragen werden, wie z.B. einem Metallmaterial, Bevorzugt wird als solches Metallmaterial Stahl oder Titan oder Legierungen daraus verwendet. Das Hammerteil kann irgendein Bauteil sein, das angetrieben auf den Arbeitskolben aufschlägt, beispielsweise ebenfalls ein Kipphebel oder eine schwenkbare Platte, deren freies Ende auf das Übertragungsstück auftrifft. Bevorzugt ist das Hammerteil als Antriebskolben ausgebildet, der in einem Antriebsrohr angetrieben beschleunigbar ist. Das Übertragungsstück ist vorteilhafterweise als Übertragungskolben ausgebildet, von dem das Antriebsrohr abgedichtet ist. Die kolbenförmige Ausbildung des Hammerteils und des Übertragungsstücks hat den Vorteil; daß die nicht in adäquater Weise sterilisierbaren antriebsseitigen Teile der Vorrichtung leicht zu dem einzutrelbenden Drahtstifthin abgedichtetwerden können, so daß dessen Kontamination in einfacher Weise verhindertwird. Der Übertragungskolben ist vorteilhafterweise langgestreckt, so daß er genügend Außenfläche zur Anordnung von Dichtelementen hat. Bevorzugt sind der Übertragungskolben und der Antriebskolben koaxial zueinanderangeordnet. Damit kann der Übertragungskolben als sich in das Antriebsrohr hineinerstreckend vorgesehen werden, so daß die Abdichtung des Antriebsrohrs weiter erleichtert ist. Insbesondere im Falle daß ein Übertragungskolben vorgesehen ist, ist dieser im axialen Abstand von der Führungshülse angeordnet, so daß eine Stoßübertragung von dem Übertragungskolben auf die Führungshülse nicht auftreten kann. Der Übertragungskolben ist ferner vorteilhafterweise in beide Axialrichtungen durch einen Anschlag begrenzt, wobei ein gewisses Axialspiel vorgesehen ist, damit der Stoß von dem Übertragungskolben nicht auf das Ihn aufnehmende Gehäuse übertragen werden kann.

Der Antrieb des Hammerteils kann insbesondere pneumatisch, hydraulisch, mechanisch oder elektromagnetisch erfolgen. Um die periodisch wiederholten Stöße auf das Übertragungsstück ausüben zu können, ist das Hammerteil periodisch hin und her bewegbar, und zwar bevorzugt über einen dervorstehend genannten Antriebe in die Schlagrichtung antreibbar und entgegen der Schlagrichtung, z.B. mittels einer Feder, selbstätig zurückstellbar.

Obwohl es beisplelsweise möglich ist, die Führungshülse zusammen mit den antriebsseitigen Teilen, wie dem Hammerteil und dem Übertragungsstück in einer einteiligen Vorrichtung vorzusehen, ist die Führungshülse bevorzugt in einem separaten Vorrichtungskopfteil vorgesehen, während das Übertragungsstück und das Hammerteil in einem Vorrichtungsantriebsteil vorgesehen sind. Das Vorrichtungskopfteil und das Vorrichtungsantriebsteil sind über eine lösbare Befestigungskupplung aneinander befestigt.

Durch diese konstruktive Trennung der antriebsseitigen Teile und der den Drahtstift haltenden und führenden Teile ist das Sterilisieren und insbesondere das Sterilhalten der mit dem einzutreibenden Draht in Kontakt kommenden Teile erheblich erleichtert. Das Vorrichtungskopfteil kann hierzu einfach von dem Vorrichtungsantriebsteil gelöst werden und ist für Reinigungswerkzeuge und Sterilisationszwecke gut zugänglich. Das Vorrichtungsantriebsteil ist als selbständige Geräteeinheit mit über das Übertragungsstück abgedichtet gekapseltem Hammerteil vorgesehen, so daß nach dem Zusammenbau des Vorrichtungskopfteils und des Vorrichtungsantriebsteils die Gefahr einer Kontamination der vorab sterilisierten Kopftellkomponenten nicht besteht.

Die erfindungsgemäße Vorrichtung ist insbesondere als Handgerät vorgesehen, soll aber trotz der demnach erforderlichen kleinen Abmessungen und Handlichkeit ausreichend stabil sein. Vorteilhafterweise ist das Vorrichtungsantriebsteil an seinem dem Vorrichtungskopftell zugewandten Endabschnitt mit einer Zwischenhülse versehen, in der das Übertragungsstück aufgenommen ist und auf die das Vorrichtungskopfteil abgedichtet aufgeschraubt oder aufgesteckt ist. Ferner hat das Vorrichtungsantriebsteil ein Gehäuse, das umfangsseitig bündig zu dem Vorrichtungskopfteil auf die Zwischenhülse aufgesteckt oder aufgeschraubt ist, und zwar insbesondere ebenfalls abgedichtet.

Die lösbare Befestigungskupplung zwischen dem Vorrichtungskopfteil und dem Vorrichtungsantriebsteil wird demnach Ober die zwischen dem Vorrichtungskopfteil und der Zwischenhülse vorliegende Verbindung erzielt. Die Zwischenhülse ermöglicht an ihrem von dem Vorrichtungskopfteil abgewandten Abschnitt das einfache zusätzliche Anbringen des die antriebsseitigen Teile umgebenden und damit schützenden Gehäuses. Es wäre allerdings auch möglich, daß das Übertragungsstück ohne Vorsehen einer Zwischenhülse direkt in einem Gehäuseteil aufgenommen ist und daß das Vorrichtungskopfteil beispielsweise direkt an diesem Gehäuseteil befestigt ist. Das Gehäuse ist zwecks einfacher Handhabung bevorzugt rohrförmig, insbesondere als kreiszylindrisches Rohr, ausgebildet. Zur besseren Abdichtung des Gehäuses bzw. des Vorrichtungskopfteils weist die Zwischenhülse vorteilhafterweise In ihrer Längsmitte einen nach außen weisenden Ringbund auf, an dem sowohl das Gehäuse als auch das Vorrichtungskopfteil anliegen. Alternativ kann zwischen dem Vorrichtungskopfteil und dem Gehäuse ein ringförmiges Dichtungselement auf der Zwischenhülse angeordnet sein, welches Dichtungselement von dem Vorrichtungskopfteil und dem Gehäuse abdichtend eingeklemmt ist.

Nach einer Ausführungsform der Erfindung weist die Vorrichtung ein Reibstück auf, welches seitlich gegen den eingesetzten Drahtstift drückt. Durch dieses Reibstück wird verhindert, daß der Drahtstift bei versehentlicher Betätigung der Vorrichtung .aus dieser wie ein Geschoß ausgestoßen wird, da eine solche unbeabsichtige Bewegung von dem Reibstück abgebremst wird. Als Reibstück kann beispielsweise die Führungshülse ausgestaltet sein, bei welcher der Drahtdurchmesser und die Führungsbohrung einander bis auf ein Reibspiel entsprechen, ohne die Längsverschiebbarkeit des Drahtstiftes in der Führungsbohrung zu dessen Einschieben in die Führungsbohrung zu verhindern. Das Reibstück ist vorzugsweise ein elastisch nachgiebiges Reibstück, beispielsweise ein Gummielement, wie ein Gummikklotz, dienen der seitlich des Drahtstifts, gegen diesen leicht vorgespannt angeordnet ist. Bevorzugt wird als Reibstück ein Dichtungselement verwendet, von dem der eingesetzte Drahtstift rings seines Umfangs gegen die Vorrichtung abgedichtet ist. Hierdurch kann die Vorrichtung zusätzlich gegen das Eindringen von Schmutzpartikeln, insbesondere Erregern, abgedichtet werden. Als Dichtungselement wird vorteilhafterweise eine Dichtungskappe aus z.B. Kunststoff eingesetzt, die am Drahtaustrittsende der Vorrichtung angeordnet ist und die ein Durchgangsloch aufweist, durch welches sich der Draht ringsum an der Kappe abgedichtet anliegend erstreckt. Mit dieser Dichtkappe ist die Vorrichtung bereits von ihrem Drahtaustrittsende her, welches das vordere Ende der Vorrichtung bildet, sicher abgedichtet.

Um zu verhindern, daß das Hammerteil seine Ausgangsposition ungewollt verläßt, ist eine Haltevorrichtung vorgesehen, mittels deren das Hammerteil in der Ausgangsposition gehalten wird. Die Haltevorrichtung kann z.B. eine Klemmvorrichtung sein, mittels deren das Hammerteil durch Klemmkräfte gehalten ist, welche von den Antriebskräften überwindbar sind. Vorteilhafterweise ist zum Halten des Hammerteils eine Magnethalterung vorgesehen, welche z.B. von einem Elektromagneten; bevorzugt aber von einem Dauermagneten gebildet ist. An dem Hammerteil kann als Gegenstück zu der Magnethalterung ein magnetisches Teil, wie ein magnetisierbares Metallteil angebracht sein. Bevorzugt ist jedoch das gesamte Hammerteil aus einem magnetisierbaren Werkstoff, wie z.B. Stahl.

Obwohl die erfindungsgemäße Vorrichtung beispielsweise zum Eintreiben eines beliebigen Drahtstifts, wie z.B. eines Nagels, in ein beliebiges Material verwendet werden kann, wird die Vorrichtung bevorzugt zum Eintreiben eines Kirschnerdrahts in Knochenmaterial verwendet. Demnach ist die Vorrichtung auch bevorzugt als medizinische Vorrichtung vorgesehen.

Die Erfindung wird nachfolgend anhand bevorzugter Ausführungsformen mit Bezugnahme auf die Zeichnung erläutert. In der Zeichnung zeigen:
Figur 1 einen Längsschnitt einer Vorrichtung zum Einbringen eines Drahtstifts in Knochenmaterial gemäß einer Ausführungsform der Erfindung und
Figur 2 eine vergrößerte Darstellung des vorderen Teils der in Figur 1 dargestellten Vorrichtung.

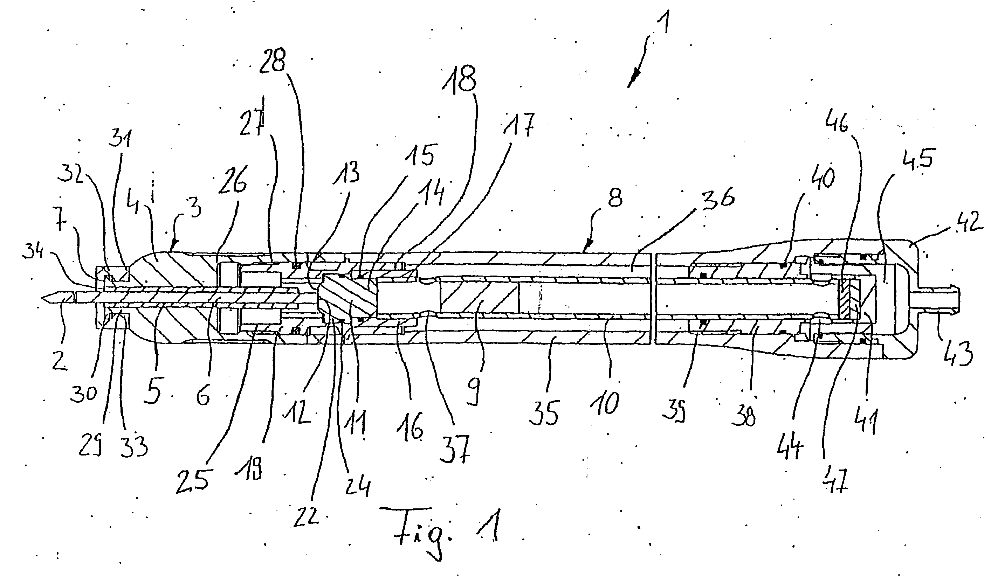

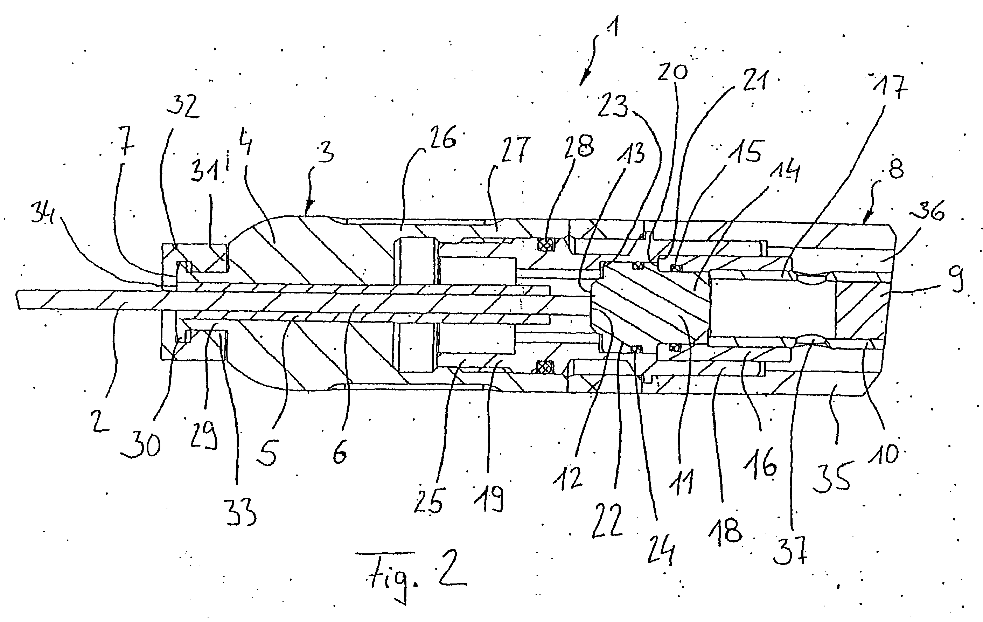

In Figuren 1 und 2 ist eine erfindungsgemäße Vorrichtung 1 zum Einbringen eines kopflosen, langgestreckten Drahtstifts, insbesondere eines Kirschnerdrahts 2, in nicht dargestelltes Knochenmaterial im Längsschnitt gezeigt. Die Vorrichtung 1 ist als Handgerät ausgebildet und weist ein Vorrichtungskopfteil 3 mit einem durchbrochenen Körper 4 und einer von diesem aufgenommenen Führungshülse 5 auf, in die der Kirschnerdraht 2 mit seinem dem Knochenmaterial abgewandten Endabschnitt 6 eingesetzt ist, so daß der langgestreckte Drahtstift oder Kirschnerdraht mit einem wesentlichen Teil seiner Länge aus der Vorrichtung herausragt.. Die Führungshülse 5 erstreckt sich ausgehend von dem dem Knochenmaterial zugewandten Stirnende 7 des durchbrochenen Körpers 4 aus fast entlang der ganzen Länge des Vorrichtungskopfteils 3. Das Vorrichtungskopfteil 3 ist, wie später noch genauer erläutert, an einem Vorrichtungsantriebsteil 8 lösbar befestigt, welches ein Hammerteil 9 in Form eines zu der Führungshülse 5 koaxial angeordneten, kreiszylindrischen Antriebskolbens aufweist, der in einem kreiszylindrischen Antriebsrohr 10 frei bewegbar aufgenommen und entlang diesem periodisch hin- und her antreibbar ist.

Das Vorrichtungsantriebsteil 8 weist ferner ein Übertragungsstück 11 auf, welches zwischen dem Hammerteil 9 und der Führungshülse 5 im axialen Abstand zu der Führungshülse 5 sowie stirnseitig an dem ihm zugewandten Stirnende des Antriebsrohrs 10 anliegend angeordnet ist. Das Übertragungsstück 11 ist kolbenförmig ausgebildet und bildet einen knochenmaterialfernen axialen Anschlag für den Kirschnerdraht 2, der hierzu, wie aus den Figuren ersichtlich ist, vom vorderen freien Vorrichtungsende her in die Vorrichtung 1 eingeschoben wird, bis der Draht mit seinem knochenmaterialfernen Stirnende 12 an dem ihm zugewandten Stirnende 13 des Übertragungsstücks 11 stumpf zur Anlage kommt. Das kolbenförmige Übertragungsstück 11 ist zu der Führungshülse 5 und damit auch zu dem Hammerteil 9 koaxial angeordnet.

Das Übertragungsstück 11 ist mit seinem dem Antriebsrohr 10 zugewandten Endabschnitt 14 radial annähernd spielfrei und damit axial geführt sowie abgedichtet in einer Kupplungshülse 16 aufgenommen, wobei die Abdichtung durch einen umfangsseitig des Übertragungsstücks 11 angeordneten O-Ring 15 erfolgt, der in einer inneren Ringnut der Kupplungshülse 16 aufgenommen ist. In der Kupplungshülse 16 ist auch das Antriebsrohr 10 mit seinem dem Übertragungsstück 11 zugewandten Endabschnitt 17 abgedichtet aufgenommen. Das Antriebsrohr 10 ist hierzu mit seinem dem Übertragungsstück 11 zugewandten Endabschnitt in die Kupplungshülse 16 gesteckt und liegt mit einem äußeren Randabschnitt seines dem Übertragungsstück 11 zugewandten Stirnendes an einer als axialer Anschlag dienenden inneren Schulter der Kupplungshülse 16 an.

Die Kupplungshülse 16 ist abgedichtet in einem von der Führungshülse 5 abgewandten Endabschnitt 18 einer Zwischenhülse 19 aufgenommen und liegt mit einem äußeren Randabschnitt ihres der Führungshülse 5 zugewandten Stirnendes 20 an einer inneren Schulter 21 der Zwischenhülse 19 an.

Das Übertragungsstück 11 weist benachbart zu seinem von der Führungshülse 5 abgewandten Endabschnitt 14 einen nach außen weisenden Ringbund 22 auf, der in der Zwischenhülse radial annähernd spielfrei aufgenommen ist und der axial von dem inneren Randabschnitt des der Führungshülse 5 zugewandten Stimendes 20 der Kupplungshülse 16 und einer inneren Anschlagschulter 23 der Zwischenhülse 19 mit geringfügigem Axialspiel begrenzt wird. Damit ist das Übertragungsstück 11 relativ zu der Zwischenhülse 19 innerhalb des vorgegebenen Axialspiels axial geringfügig bewegbar. In dem Ringbund 22 des Übertragungsstücks 11 ist umfangsseitig eine Ringnut ausgebildet, in der ein O-Ring 24 angeordnet ist, von dem das Übertragungsstück 11 umfangsseitig gegen die Zwischenhülse 19 abgedichtet ist.

Die Zwischenhülse 19 ist an ihrem dem Vorrichtungskopfteil 3 zugewandten Endabschnitt 25 mit einem axialen Außengewinde versehen. An dem durchbrochenen Körper 4 ist an seinem dem Vorrichtungsantriebsteil B zugewandten Endabschnitt 26 ein Ringflansch 27 mit einem axialen Innengewinde angeformt, über den der durchbrochene Körper 4 und damit das Vorrichtungskopfteil 3 auf den ihm zugewandten Endabschnitt 26 der Zwischenhülse 19 abgedichtet aufgeschraubt ist. Die Abdichtung erfolgt über einen umfangsseitig der Zwischenhülse 19 angeordneten O-Ring 28, der in einer in der Zwischenhülse 19 ausgebildeten Ringnut angeordnet ist.

Der im Gebrauch der Vorrichtung 1 dem Knochenmaterial zugewandte Endabschnitt 29 des durchbrochenen Körpers 4 ist unter Ausbildung eines stimendeseitigen Kragens 30 mit einer äußeren Ringnut 31 versehen. Auf diesen Endabschnitt 29 des durchbrochenen Körpers 4 ist eine durchbrochene Kappe 32 aus elastischem Material aufgesetzt, die mit einem in die Ringnut 31 eingreifenden, nach innen weisenden Ringbund 33 versehen ist. Der Durchbruch in der Verschlußkappe 32 ist derart, daß sich der in die Führungshülse 5 eingesetzte Kirschnerdraht 2 umfangsseitig an die Verschlußkappe 32 anliegend durch diese erstreckt. Die Verschlußkappe 32 liegt ferner sowohl an dem Stirnende 7 des durchbrochenen Körpers 4 als auch an dem Stirnende 34 der Führungshülse 5 an.

Das Vorrichtungsantriebsteil B weist ein als Gehäuse der antriebsseitigen Teile dienendes kreiszylindrisches Aufnahmerohr 35 auf, das auf den von dem Vorrichtungskopftell 3 abgewandten Endabschnitt 1B der Zwischenhülse 19 abgedichtet aufgesteckt ist und in dem das Antriebsrohr 10 aufgenommen ist. Zwischen dem Aufnahmerohr 35 und dem Antriebsrohr 10 ist eine Ringkammer 36 ausgebildet, die über benachbart zu der Kupplungshülse 16 in dem Antriebsrohr 10 seitlich ausgebildete Öffnungen 37 mit dem Inneren des Antriebsrohrs 10 in Verbindung steht.

Die Ringkammer 36 wird an ihrem von dem Vorrichtungskopfteil 3 abgewandten Ende. axial von einer Dichthülse 38 begrenzt, die das Antriebsrohr 10 umgebend axial in das Aufnahmerohr 35 geschraubt ist und mittels O-Ringen 39, 40 gegen das Antriebsrohr 10 und das Aufnahmerohr 35 abgedichtet ist.

Das von dem Vorrichtungskopfteil 3 abgewandte Ende des Antriebsrohrs 10 ist mit einem Verschlußdeckel 41 dicht verschlossen, der gleichzeitig einen übertragungsstückfernen Anschlag für das Hammerteil 9 bildet. Das von dem Vorrichtungskopfteil 3 abgewandte Ende des Aufnahmerohrs 35 ist mit einem Stopfen 42 dicht verschlossen, an dem ein Anschlußstutzen 43 für die Zuführung von Preßluft ausgebildet ist. Das Antriebsrohr 10 ist benachbart zu dem Verschlußdeckel 41 mit seitlichen Öffnungen 44 versehen, die über eine zwischen dem Stopfen 42, der Dichthülse 38, dem Aufnahmerohr 35 und dem Antriebsrohr 10 ausgebildete Kammer 45 mit der Öffnung des Stutzens 43 in Verbindung stehen. In dem Verschlußdeckel 41 sind eine starre Platte 46 und eine elastische Platte 47 angeordnet, die zusammen als axialer, gepufferter Anschlag für das Hammerteil 9 dienen. Hierzu sind die beiden Platten 46, 47 unmittelbar hintereinander angeordnet, wobei die starre Platte 46 zu dem Hammerteil 9 näher liegt. Die elastische Platte 47 ist aus einem elastischen Kunststoff oder aus Gummi und dient als Puffer. Die starre Platte 46 ist aus einem dauermagnetischen Material, d.h. die Platte 46 bildet einen Dauermagneten. Das Hammerteil 9 ist aus einem magnetisierbaren Material, wie z.B. Stahl. Damit bildet die starre Platte 46 für das Hammerteil 9 eine Magnethalterung, an der das Hammerteil 9 in seiner Ausgangsposition gegen ungewolites Bewegen gesichert gehalten ist. Die magnetischen Kräfte zwischen der starren Platte 46 und dem Hammerteil 9 sind so bemessen, daß sie von den Antriebskräften überwunden werden.

Im folgenden wird die Funktionsweise der erfindungsgemäßen Vorrichtung 1 zum Einbringen eines Kirschnerdrahts 2 erläutert. Zunächst wird der Kirschnerdraht 2 in die Führungshülse 5 in dem Vorrichtungskopfteil 3 bis zum stirnseitigen Anschlagen an dem Übertragungsstück 11 eingesetzt. Die Vorrichtung 1 wird dann mit dem dem Knochenmaterial zugewandten Ende des Kirschnerdrahts 2 gegen das Knochenmaterial gedrückt. Hierdurch ist gewährleistet, daß das Übertragungsstück 11 mit seinem Ringbund 22 gegen die Kupplungshülse 16 gedrückt ist, wodurch auch das zur einwandfreien Stoßübertragung auf den Kirschnerdraht 2 erforderliche Axialspiel zwischen dem Ringbund 22 und der vorrichtungskopfteilseitigen Anschlagschulter 23 der Zwlschenhülse 19 gewährleistet ist.

Zum Eintreiben des Kirschnerdrahts 2 in das Knochenmaterial wird der Vorrichtung 1 über den Stutzen 43 periodisch Druckluft zugeführt. Die zugeführte Druckluft gelangt durch die Kammer 45 und die Öffnungen 44 in das Antriebsrohr 10 und beschleunigt das Hammertell 9 in den Figuren nach links in Richtung zu dem Übertragungsstück 11. Die von dem Hammerteil 9 in dem Antriebsrohr 10 verdrängte Luftwird über die Öffnungen 37 in die Ringkammer 36 verdrängt. Das Hammerteil 9 wird bis zum Anschlagen an dem Übertragungsstück 11 mit über den Stutzen 43 zugeführter Druckluft beaufschlagt. Nach dem Aufschlagen auf das Übertragungsstück 11 wird das Hammerteil 9 von der in der Ringkammer 36 komprimierten Luft, die über die Öffnungen 37 zurück in das Antriebsrohr 10 strömt, zurück bis an den von dem Verschlußdeckel 41 gebildeten Anschlag bewegt. Anschließend wird über den Stutzen 43 wieder Preßluft zugeführt, so daß sich der vorstehend erläuterte Antriebsvorgang wiederholt.

Mit jedem Aufschlagen des Hammerteils 9 auf das Übertragungsstück 11 wird auf dieses ein Stoßimpuls übertragen und auf den an dem Übertragungsstück 11 anliegenden Kirschnerdraht 2 weiterübertragen. Bevorzugt wird in dem Übertragungsstück 11 vor allem eine Kompressionswelle angeregt und auf den Kirschnerdraht 2 übertragen. Durch das Axialspiel zwischen dem Ringbund 22 des Übertragungsstücks 11 und der inneren Anschlagschulter 23 des Zwischenhülse 19 wird verhindert, daß der Stoß auf die Zwischenhülse 19 übertragen wird. Durch das Axialspiel zwischen dem Übertragungsstück 11 und der den Kirschnerdraht 2 aufnehmenden Führungshülse 5 ist gewährieistet, daß der Stoßimpuls vollständig auf den Kirschnerdraht 2 und nicht auf die Führungshülse 5 übertragen wird. Die langgestreckte Führungshülse 5 bewirkt, daß der Kirschnerdraht 2 in seinem dem Übertragungsstück 11 zugewandten Endabschnitt 6 ungekrümmt ist, wodurch sich andernfalls bei der Stoßübertragung ein mit einem Energieverlust verbundenes Verbiegen des Drahts 2 in diesem Endabschnitt ergeben kann. Der Stoßimpuls oder auch die Kompressionswelle pflanzt sich in dem Kirschnerdraht 2 fort und bewirkt ein Auslenken dessen knochenmaterialseitigen Endes in das Knochenmaterial. Das in das Knochenmaterial hinein ausgelenkte Ende des Kirschnerdrahts 2 bleibt in dem Knochenmaterial haften, da der an diesem Drahtende reflektierte Stoßimpuls stark abgeschwächt ist und das Drahtende nicht mehr aus dem Knochenmaterial herausverlagern kann. Der restliche Abschnitt des Kirschnerdrahts 2 wird durch dieses Steckenbleiben seines Drahtendes automatisch nachgeführt. Damit entsteht zwischen dem knochenmaterialfemen Stirnende 12 des Kirschnerdrahts 2 und dem Stirnende 13 des Übertragungsstücks 11 ein kleine Lücke, die aber durch das stetige Drücken der ganzen Vorrichtung 1 in die Bewegungsrichtung des Kirschnerdrahts 2 sofort geschlossen wird, so daß dieser mit seinem Stirnende 12 stets an dem Übertragungsstück 11 anliegt.

Damit wird der Kirschnerdraht 2 bei ständigem Nachführen der Vorrichtung 1 in die Eintreibrichtung des Drahts 2 durch In-Kontakt-Halten des Übertragungsstücks 11 mit dem ihm zugewandten Stirnende 12 des Kirschnerdrahts 2 sowie durch periodisch wiederholtes Anschlagen des Hammerteils 9 gegen das Übertragungsstück 11 in das Knochenmaterial eingetrieben.

Der Verschlußkappe 32 kommt eine Doppelfunktion zu. Einerseits dichtet sie die Vorrichtung 1 gegen den Kirschnerdraht 2 ab, andererseits bildet sie eine Reibstück, welches an dem Kirschnerdraht anliegt, so daß dieser bei einer versehentlichen Betätigung der Vorrichtung 1 nicht aus dieser herausschießen und evtl. ein nebenstehende Person verletzten kann, da die translatorische Bewegung des Drahts von der daran reibend anliegenden Kappe 32 gebremst wird.

## Patentansprüche

1. Kombination aus einer Vorrichtung (1) zum Eintreiben eines langgestreckten Drahtstifts, insbesondere eines Kirschnerdrahts (2), in ein Knochenmaterial, und dem langgestreckten Drahtstift, die Vorrichtung versehen mit einer an dem Drahtstift, der in die Vorrichtung (1) vom vorderen freien Vorrichtungsende her einschiebbar ist, angreifenden Vortriebsvorrichtung, die von einem in periodischer Wiederholung Anschlagstöße erzeugenden Hammerteil (9) angetrieben ist, das seinerseits periodisch angetrieben ist, wobei die Vortriebsvorrichtung an dem eingeschobenen Drahtstift klemmittelfrei angreift und die Vortriebsvorrichtung als elastische Stoßwellen übertragendes Übertragungsstück (11) ausgebildet ist, an dem der eingeschobene Drahtstift mit seinem freien Stirnende stumpf anliegt und von dem die Anschlagstöße des Hammerteils (9) in Form von periodischen Stoßimpulsen durch die freie Stirnfläche des Drahtstifts hindurch in diesen einleitbar sind, so daß sich die Stoßimpulse in dem Drahtstift jeweils in Form einer Kompressionswelle unter periodisch fortschreitendem Eintreiben des aus dem vorderen freien Vorrichtungsende herausragenden Abschnittes des Drahtstiftes in das Knochenmaterial und entsprechendem Nachführen des restlichen Abschnitts des Drahtstiftes aus der Vorrichtung fortpflanzen, und mit einer Führungshülse (5), in welcher der Drahtstift bis zum Anschlagen an dem Übertragungsstück (11) längsverschiebbar aufgenommen und geführt ist und welche mit dem Übertragungsstück (11) nicht in stoßübertragendem Kontakt steht.

2. Kombination (1) nach Anspruch 1, wobei das Hammerteil (9) als Antriebskolben ausgebildet ist, der in einem Antriebsrohr (10) periodisch angetrieben beschleunigbar ist, und wobei das Übertragungsstück (11) als Übertragungskolben ausgebildet ist, von dem das Antriebsrohr (10) abgedichtet ist.

3. Kombination (1) nach Anspruch 1 oder 2, wobei die Führungshülse (5) in einem Vorrichtungskopfteil (3) vorgesehen ist, wobei das Übertragungsstück (11) und das Hammerteil (9) in einem Vorrichtungsantriebsteil (8) vorgesehen sind, und wobei das Vorrichtungskopfteil (3) und das Vorrichtungsantriebsteil (8) über eine lösbare Befestigungskupplung aneinander befestigt sind.

4. Kombination (1) nach Anspruch 3, wobei das Vorrichtungsantriebsteil (8) an seinem dem Vorrichtungskopfteil (3) zugewandten Endabschnitt eine Zwischenhülse (19) aufweist, in der das Übertragungsstück (11) aufgenommen ist und auf die das Vorrichtungskopfteil (3) abgedichtet aufgeschraubt ist, und wobei das Vorrichtungsantriebsteil (8) ein Gehäuse (35) aufweist, das umfangsseitig bündig zu dem Vorrichtungskopfteil (3) auf die Zwischenhülse (19) aufgesteckt ist.

5. Kombination (1) nach einem der Ansprüche 1 bis 4, ferner mit einem Reibstück, welches seitlich gegen den eingesetzten Drahtstift gedrückt ist.

6. Kombination nach Anspruch 5, wobei das elastisch nachgiebig ausgebildete Reibstück als Dichtungselement ausgebildet ist, von dem der eingesetzte Drahtstift rings seines Umfangs abgedichtet ist.

7. Kombination nach Anspruch 6, wobei das Dichtungselement als Dichtungskappe ausgebildet ist, die am Drahtaustrittsende der Vorrichtung angeordnet ist, durch welche sich der Drahtstift abgedichtet hindurcherstreckt.

8. Kombination nach einem der Ansprüche 1 bis 7, wobei die Führungshülse (5) axial im Abstand von dem Übertragungsstück (11) angeordnet ist.

## Claims

1. A combination of a device (1) for driving an elongated wire pin, in particular a Kirschner-pin (2), into bone material, and the elongated wire pin, the device comprising a propelling device that acts upon the wire pin which is insertable into the device (1) from the front free end thereof, said propelling device being driven by periodically repeated impact strokes applied thereupon by a periodically driven impacting member (9), wherein the propelling device acts upon the inserted wire pin without any clamping means being involved, and wherein the propelling device is designed as a transmitting member (11) which transmits elastic impact waves and against which the free end face of the inserted wire pin evenly abuts, with the transmitting member being capable of transmitting the impact strokes of the impacting member (9) as periodical impact pulses into the wire pin through the free end face thereof so that each impact pulse propagates through the wire pin in the form of a compression wave, whereby the wire pin protruding from the free front end of the device is periodically and progressively driven into the bone material and the remaining part of the wire pin is operated thereafter out of the device, respectively, and comprising a guiding sleeve (5) which is not in impact transmitting contact with the transmitting member (11), wherein the wire pin is guided and received so that it is movable in the longitudinal direction until it abuts against the transmitting member (11).

2. The combination (1) according to claim 1, wherein the impacting member (9) being designed as a drive piston which may be periodically accelerated in an drive pipe (10), and wherein the transmitting member (11) being formed as a transmitting piston which seals the drive pipe (10).

3. The combination (1) according to claim 1 or 2, wherein the guiding sleeve (5) is provided in a head unit (3) of the device, and the transmitting member (11) and the impacting member (9) are provided in a drive unit (8) of the device, and the head unit (3) and the drive unit (8) are mounted to each other by a separable mounting coupling.

4. The combination (1) according to claim 3, wherein the drive unit (8) comprises an intermediary sleeve (19) at its end portion facing the head unit (3), in which the transmitting member (11) is received and onto which the head unit (3) is threaded in a sealed manner, and wherein the drive unit (8) comprising a casing (35) which is attached to the intermediary sleeve (19) so that it is circumferentially flush with the head unit (3).

5. The combination (1) according to any one of claims 1 to 4, further with a friction member which is pressed against the lateral surface of the inserted wire pin.

6. The combination (1) according to claim 5, wherein the friction member is an elastic friction member and is formed as a sealing member which sealingly surrounds the circumference of the inserted wire pin.

7. The combination (1) according to claim 6, wherein the sealing member is formed as a sealing cap which is arranged at the wire-outlet end of the device and through which the wire pin extends in a sealed manner.

8. The combination (1) according to any one of claims 1 to 7, wherein the guiding sleeve (5) is arranged at an axial distance from the transmitting member (11).

## Revendications

1. Combinaison d'un dispositif (1) pour enfoncer une pointe allongée, en particulier un fil de Kirschner (2), dans un matériau osseux, et la pointe allongée, le dispositif étant muni d'un dispositif d'avancement s'engageant sur la pointe, qui peut être insérée dans le dispositif (1) par l'extrémité avant libre du dispositif, ledit dispositif d'avancement étant entraîné par une partie formant marteau (9) produisant des impacts répétés périodiquement, qui est entraînée périodiquement pour sa part, le dispositif d'avancement s'engageant sur la pointe insérée sans moyen de serrage et le dispositif d'avancement se présentant sous la forme d'une pièce de transfert (11) transférant des ondes de choc élastiques, sur lequel la pointe insérée s'applique bout à bout par son extrémité avant libre et par laquelle les impacts de la partie formant marteau (9), sous la forme d'impulsions de choc périodiques, peuvent être acheminés via la surface avant libre de la pointe dans celle-ci, de telle sorte que les impulsions d'impact se propagent dans la pointe respectivement sous la forme d'une onde de compression en enfonçant progressivement et périodiquement la partie de la pointe dépassant de l'extrémité avant libre du dispositif dans le matériau osseux et en guidant de manière correspondante la partie restante de la pointe hors du dispositif, et avec une douille de guidage (5), dans laquelle la pointe est reçue et guidée de manière à pouvoir se déplacer longitudinalement jusqu'à venir buter sur la pièce de transfert (11), et qui n'est pas en contact de transfert de choc avec la pièce de transfert.

2. Combinaison (1) selon la revendication 1, dans laquelle la partie formant marteau (9) se présente sous la forme d'un piston d'entraînement, qui peut être accéléré en étant entraîné périodiquement dans un tube d'entraînement (10), et dans lequel la pièce de transfert (11) se présente sous la forme d'un piston de transfert par rapport auquel le tube d'entraînement (10) est étanché.

3. Combinaison (1) selon la revendication 1 ou 2, **caractérisée en ce que** la douille de guidage (5) est ménagée dans une partie de tête (3) du dispositif, **en ce que** la pièce de transfert (11) et la partie formant marteau (9) sont ménagées dans une partie (8) d'entraînement du dispositif, et **en ce que** la partie de tête (3) du dispositif et la partie (8) d'entraînement du dispositif sont fixées l'une à l'autre par le biais d'un couplage de fixation libérable.

4. Combinaison (1) selon la revendication (3), **caractérisée en ce que** la partie (8) d'entraînement du dispositif présente sur sa section d'extrémité tournée vers la partie de tête (3) du dispositif une douille intermédiaire (19), dans laquelle la pièce de transfert (11) est reçue et sur laquelle la partie de tête (3) du dispositif est vissée de manière étanche, et dans lequel la partie (8) d'entraînement du dispositif présente un boîtier (35) qui est emboîté côté périphérique à fleur avec la partie de tête (3) du dispositif sur la douille intermédiaire (19).

5. Combinaison (1) selon l'une quelconque des revendications 1 à 4, **caractérisée par** une pièce de frottement, qui est pressée latéralement contre la pointe insérée.

6. Combinaison selon la revendication 5, dans laquelle la pièce de frottement formée de manière souple et élastique se présente sous la forme d'un élément d'étanchéité par lequel la pointe insérée est étanchée sur sa périphérie.

7. Combinaison selon la revendication 6, dans laquelle l'élément d'étanchéité se présente sous la forme d'un chapeau d'étanchéité, qui est agencé à l'extrémité de sortie de la pointe du dispositif, à travers laquelle la pointe s'étend de manière étanche.

8. Combinaison selon l'une quelconque des revendications 1 à 7, dans laquelle la douille de guidage (5) est agencée axialement à distance de la pièce de transfert (11).
